# EUROPEAN PATENT APPLICATION

(11) **EP 3 069 722 A1**
(43) Date of publication of application: **21.09.2016**
(21) Application number: 16164633.6
(22) Date of filing: 27.02.2004
(51) Int. Cl.: A61K 31/7032, A61K 35/12, A61K 38/17, A61P 35/00, A61P 31/00, A61P 37/02, A61K 38/00, A61K 31/00, A61K 31/7004, A61K 31/7028, A61K 35/15, A61K 35/17

(54) **REGULATION OF IMMUNE RESPONSES BY MANIPULATION OF INTERMEDIARY METABOLITE LEVELS**

(30) Priority: 27.02.2003 US 375906
(62) Divisional of application: 04004583.3
(71) Applicant: ENZO THERAPEUTICS, INC., Farmingdale, New York 11735-4716 (US)
(72) Inventor: ILAN, Yaron, 96400 Jerusalem (IL); ENGELHARDT, Dean L., New York, NY New York 10024 (US); ZIMRAN, Ari, 91031 Jerusalem (IL); MARGALIT, Maya, 68033 Jaffa (IL); RABBANI, Elazar, New York, NY New York 10003 (US)
(74) Representative: Vossius & Partner Patentanwälte Rechtsanwälte mbB

(57) **Abstract**

The present invention provides novel processes for regulating immune responses in mammalian subjects, e.g., humans, afflicted with diseases such as cancers, infections, e.g., viral infections, bacterial infections, or immune dysfunctions, especially auto-immune disorders, e.g., diabetes, Crohn's disease, rheumatoid arthritis, arteriosclerosis and ulcerative colitis. More particularly, this invention relates to generating elevated levels of an intermediary metabolite, e.g., lipids or conjugated biomolecules, e.g., glycolipids, lipoproteins and glycoproteins other than antibodies, cytokines or hormones. Treatment can be carried by introduction of the intermediary metabolite into the afflicted subject or by a reagent that when administered leads to elevated levels. The treatment regimen can be in vivo or ex vivo.

## Description

### FIELD OF THE INVENTION

This invention relates to processes for regulating and manipulating immune responses in mammalian subjects. More particularly, it relates to immune response regulation by manipulating the levels of intermediary metabolite levels. These processes can be usefully applied to the treatment of immune related or immune mediated diseases or disorders, treatment of infected subjects or treatment of cancer.

All patents, patent applications, patent publications, scientific articles and the like, cited or identified in this application are hereby incorporated by reference in their entirety in order to describe more fully the state of the art to which the present invention pertains.

### BACKGROUND OF THE INVENTION

There are a large number of genetic diseases that have been recently characterized having defective gene products that are involved in a metabolic pathway. These defects have had the twin repercussions of limiting the availability of the normal end product of such a pathway and a buildup of the intermediate whose further processing has been reduced or eliminated. Although usually detrimental and often fatal to the individual manifesting this loss or reduction of function, it has opened windows into understanding multiple effects that ensue from blockage of such pathways. One such genetic disease is Gaucher's Disease where there is a buildup of glucosylcerebroside due to a decreased capacity for breakdown of this product. Numerous mutation sites have been located that are responsible for defects in glucosylcerebrosidase activity with varying degrees of expression of the disease state. Gaucher's Disease is currently classified as Class I, II or III depending upon the particular expression phenotype of the disease.

Although this is a lipid storage or processing disease that is involved in buildup of an intermediary metabolite, a notable presentation of this disease is a defect in the immune system as well. Indeed, one of the earlier paper describing the syndrome was titled "Gaucher's Disease: a disease with chronic stimulation of the immune system" (Schoenfeld et al., 1982 Arch Pathol Lab Med 106; 388-391). As the immune system has been investigated, a large number of cytokines have been discovered that are intimately involved in promoting or repressing immune activity. Recent papers have shown that the serum levels of some cytokines a well as other components of the immune system are significantly changed in Gaucher's Disease patients. These have included IL-1β, IL-1Ra, IL-2R, IL-6, IL-8, IL-10, M-CSF, sCD14, TNF-α, gammaglobulins and β2 microglobulin (Lichtenstein et al. 1997 Blood Cells, Molecules and Diseases 23; 395-401, Barak et al., 1997 Eur Cytokine Network 10; 205-210, Hollak et al., 1997 Blood Cells, Molecules and Diseases 23; 201-212, Allen et al., 1987 Q J Med 90; 19-25, Deibener et al., 1998 Haematologica 1998 83; 479-480). It should be pointed out that these studies have not demonstrated a uniformity of response of these markers, but it has been long known that expression of Gaucher's disease is phenotypically very variable.

This effect on immune markers can be directly linked to the buildup in levels of glucosylcerebroside. For instance, some of the studies cited previously have included the effects noted both before and after treatment of patients with alglucerase. In these studies it has been discovered that after this treatment, many of the immune activation markers that were abnormally high were returned to more normal levels. Furthermore, an early study of direct application of glucosylerebroside to macrophages growing in culture resulted in the elicitation of sercretion of IL-1. It is further noted that a paper by Lachmann et al. (Timothy Cox's group in Cambridge) (QJM 2000; 93:237-244), in which four patients with massive hepatic fibrosis are described, emphasized that the development of cirrhosis in patients with Gaucher's disease is rare, usually occurring in patients who underwent splenectomy. As many untreated Gaucher patients have hepatomegaly and approximately 20% also have elevated (particularly hepatocellular) LFTs, it seems that something is protecting these patients from developing cirrhosis.

Stimulation of immune system has also been seen by introduction of α-glucosylcerabroside (Kawano et al., 1997 science 278; 1626-1629, Burdin et al., 1998 J. Immunol 161; 3271-3281). This is apparently an antigen-induced series of events since this compound was isolated from a marine sponge and is not a compound normally found in mammalian cells.

### SUMMARY OF THE INVENTION

This invention relates to processes for regulating and manipulating immune responses in mammalian subjects. More particularly, it relates to immune response regulation by manipulating the levels of intermediary metabolite levels. These processes can be usefully applied to the treatment of immune related or immune mediated diseases or disorders, treatment of infected subjects or treatment of cancer. In a preferred embodiment, such mammalian subjects are human beings.

This invention provides a process for treating a disease in a mammalian subject comprising administering to the subject an effective amount of a mammalian intermediary metabolite so as to raise the intracellular or extracellular or serum level of the metabolite in the subject.

This invention further provides a process for treating a disease in a mammalian subject comprising administering to the subject an effective amount of a reagent that increases the intracellular or extracellular or serum level of a mammalian intermediary metabolite in the subject.

The present invention also provides an ex vivo process for treating a disease in a mammalian subject. In this process, cells are first obtained from the subject. The removed cells are then treated with an effective amount of a mammalian intermediary metabolite so as to raise the intracellular level of the metabolite in the cells. Afterwards, the treated cells are transferred to the subject.

Another aspect of the present invention concerns a process for treating a disease in a mammalian subject. In the first step, cells are obtained from the subject and then treated ex vivo with an effective amount of a reagent that increases the intracellular level of a mammalian intermediary metabolite in the cells. The treated cells are then transferred to the subject.

Yet another aspect of this invention involves a process for treating a disease in a mammalian subject. Here, an effective amount of a mammalian metabolite is administered to the subject so as to modulate or change at least one component in the immune system of the subject.

Numerous other aspects and embodiments of the present invention are described in further detail below.

### BRIEF DESCRIPTION OF THE FIGURES

FIGURE 1 shows the effect of Gaucher's Disease and HCV infection on HCV specific T-cell proliferation assay.
FIGURE 2 shows the effect of Gaucher's Disease and HCV infection on HCV specific IFNγ ELISPOT assay.
FIGURE 3 shows the effect of Gaucher's Disease and HCV infection on HCV specific IL-10 ELISPOT assay.
FIGURE 4 shows the effect of Gaucher's Disease and HCV infection on IFNγ serum levels.
FIGURE 5 shows the effect of Gaucher's Disease and HCV infection on IL-4 serum levels.
FIGURE 6 shows the effect of Gaucher's Disease and HCV infection on peripheral NKT lymphocytes.

### DETAILED DESCRIPTION OF THE INVENTION

The present invention provides processes for regulation or manipulation of the immune system of a mammalian subject by altering the intracellular or serum levels of intermediate metabolites in said subject. Such process may provide at least one change in one or more components of the immune system in said subject and such a change can be specific to a particular antigen or set of antigens or it may be of a general nature or it may even comprise both of these effects. Such a manipulation or change in the immune response may be achieved directly or indirectly. Direct means can include introduction of the metabolite into the subject. Indirect means can include increasing the rate of synthesis of said metabolite or by inhibiting in vivo degradation of said metabolite in said subject. Alternatively to such *in vivo* treatments, immune cells can be treated *ex vivo* and reintroduced into the subject. The processes of the present invention may be used for treatment of immune related or immune mediated diseases or disorders. These processes may also be used for treatment of cancer subjects or infected subjects. See U.S Patent Application Serial No. 08/808,629, filed on filed on February 28, 1997; see also U.S. Patent Application 09/356294, filed on July 16, 1999; and European Patent Application No. 00 11 4901.2 (EP 107 227 A1), filed on July 17, 2000. The foregoing disclosures are hereby incorporated by reference and may further be used in conjunction with the present invention.

In the present invention, metabolites or intermediary metabolites are considered to be products of enzymatic processes in a mammalian system. Such processes can include enzymatic synthesis, enzymatic degradation, enzymatic modification. Such products may include but not be limited to lipids, saccharides, glycoplipids, lipoproteins, and glycoproteins other than antibodies, cytokines or hormones. Such products may be produced in a mammalian system, a non-mammalian system, produced through recombinant DNA, produced in vitro, created synthetically or any combination thereof. Furthermore, such elevated levels of metabolites could also be obtained in the subject indirectly either through enhancement of synthesis of the compound or inhibition of degradation of the compound. Examples of means for enhancement could include introduction of a precursor, an enzyme responsible for its synthesis or both. Examples of means for Inhibition could include introduction of an inhibitor of a degradative enzyme or antisense inhibition. Examples of glycoproteins that may be useful in the presnent invention can include but be limited to glycosylated. There may be other enzymes in the metabolic pathway which when present in elevated levels that may participate in immune modulation An example of this could be chiotriosidase which has been seen to be elevated in Gaucher's patients (Hollak et al., 1994 J. Clin Invest. 93; 1288-1292).

It is another aspect of the present invention that modulation of at least one component of the immune system or treatment of a subject with an infection or cancer may be achieved through the principles and procedures described herein, including ex vivo processes.

Distal regulation of metabolic and cellular processes are governed by hormonal signals. The role of the immune system has previously been perceived to be limited primarily to providing protection against foreign agents or foreign compounds. This effect may be accompanied by incidental interactions with self (for example auto-immune disorders) that produce deleterious effects. In addition to such immune surveillance of foreign substance, the immune system may also be engaged in surveillance of metabolic processes. As such, the immune response would recognize aberrant levels of some class or classes of metabolites providing a feedback process between the immune system and aberrant levels. As such these metabolites may provide signals and act as immune messengers. An analogous system is cyclic AMP which is known to be capable of effecting a large number of different metabolic processes.

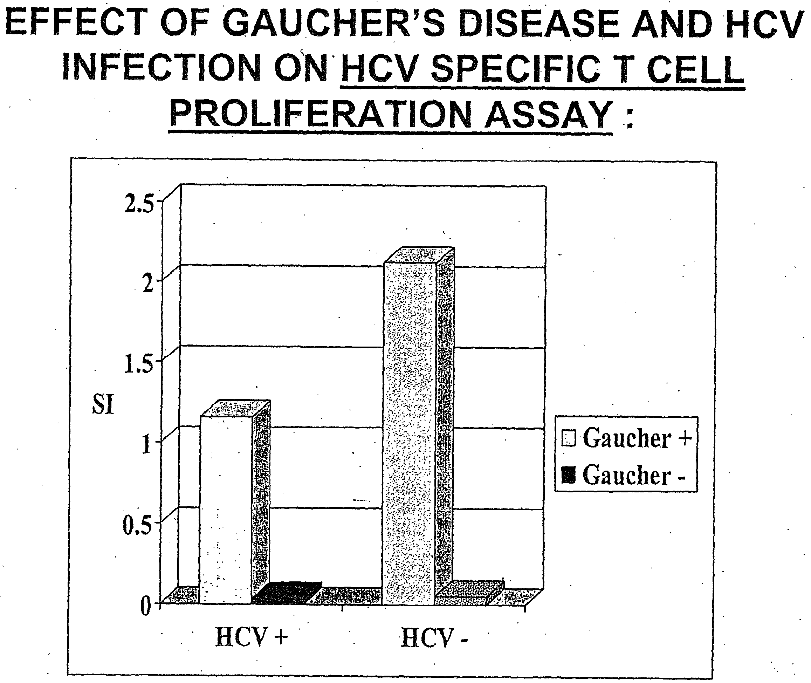

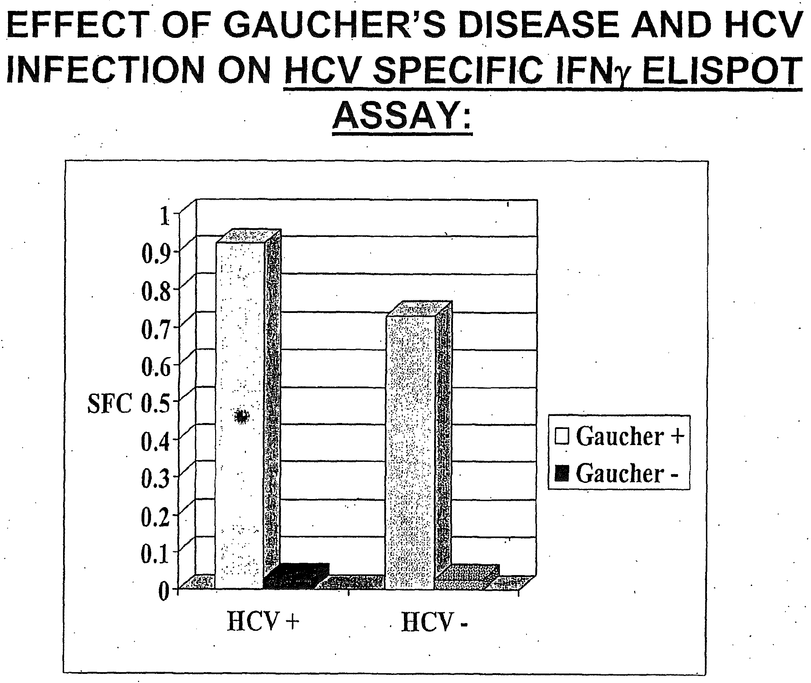

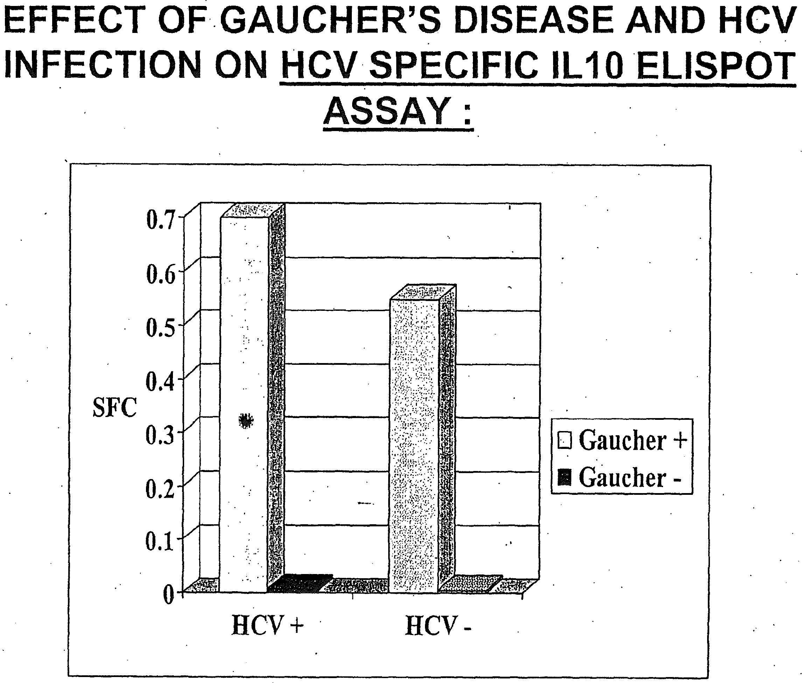

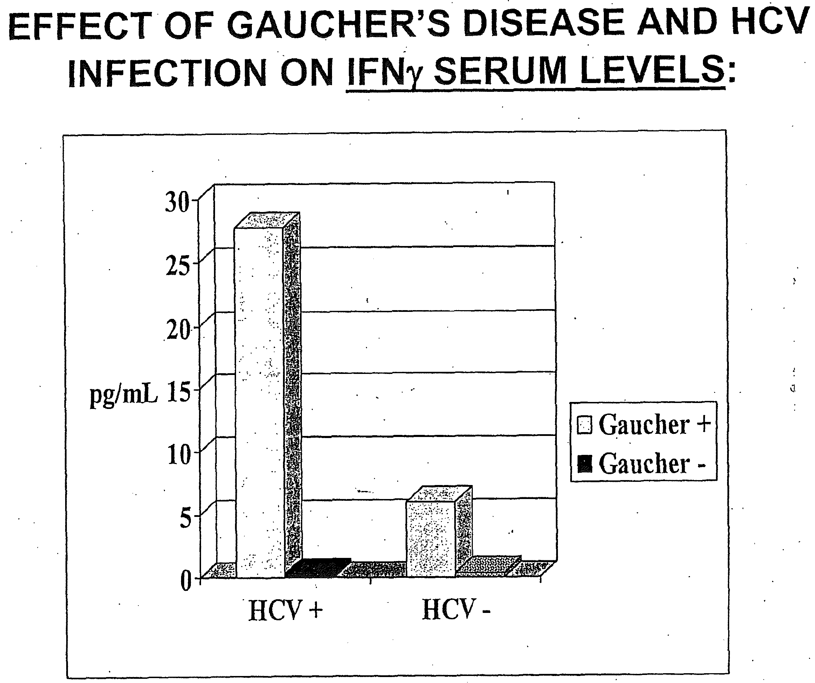

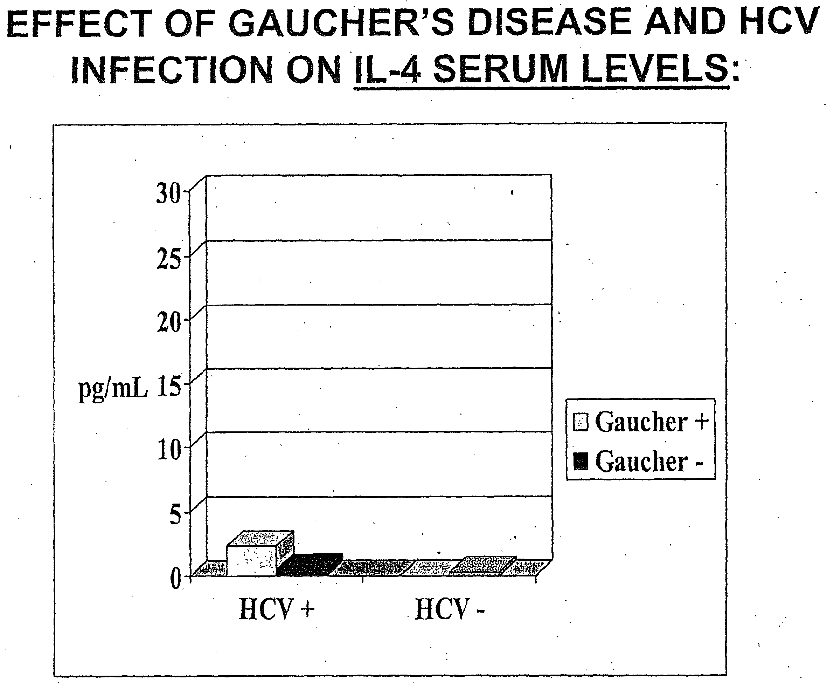

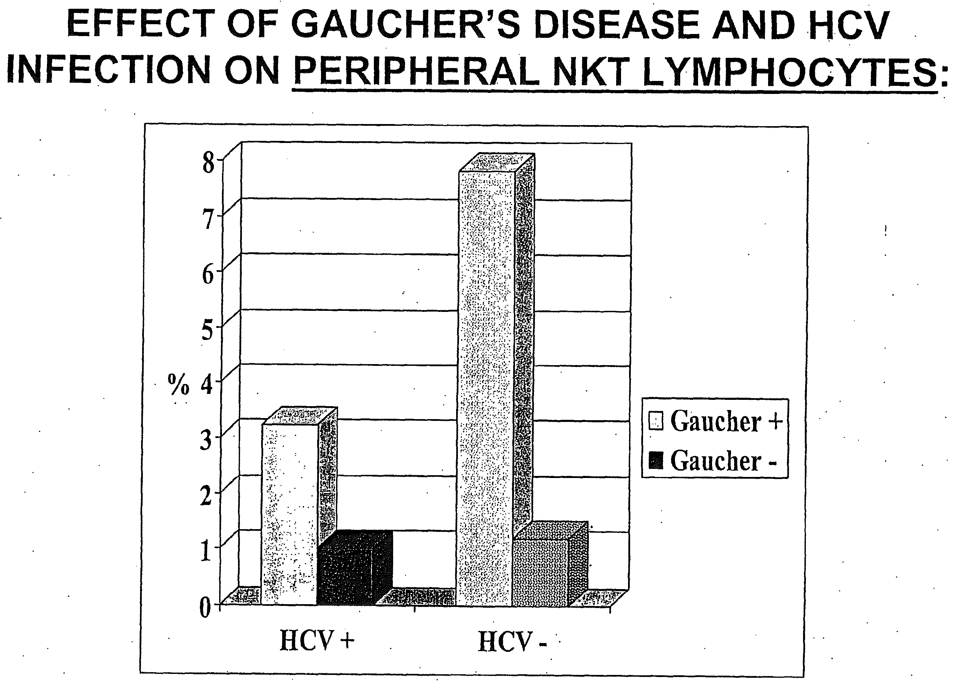

Support for such interactions has emerged from unexpected results where an aberrant immune state was derived from superimposition of two different processes that can indepently affect the immune system. One process was infection by HCV, which is an agent that conveys both immunosuppressive as well as immunoreactive responses to an infected subject (Ferrari C, Urbani S, Penna A, Cavalli A, Valli A, Lamonaca V, Bertoni R, Boni C, Barbieri K, Uggeri J, Fiaccadori F. Immunopathogenesis of hepatitis C virus infection. J Hepatol. 1999; 31 Suppl 1:31-8; Cerny A, Chisari FV. Pathogenesis of chronic hepatitis C: immunological features of hepatic injury and viral persistence. Hepatology. 1999 Sep;30(3):595-601; and Rehermann B. Cellular immune response to the hepatitis C virus. J Viral. Hepat. 1999 Jul;6 Suppl 1:31-5). The other process was Gaucher's disease which as discussed previously has many immune aberrations as a result of elevated levels of an intermediary metabolite. These surprising results were derived from analysis of a group which included Gaucher patients with (n = 5) and without (n = 17) HCV infection, non-Gaucher patients with chronic HCV (n = 15) and naïve controls (n =11). Results from these patients are shown in Figures 1 through 6. These assays include HCV specific T cell proliferation (Figure 1); HCV specific IFNγ ELISPOT (Figure 2); HCV specific IL-10 ELISPOT (Figure 3); IFNγ serum levels (Figure 4); IL-4 serum levels (Figure 5); and peripheral NKT lymphocyte measurements (Figure 6).

### Methods for carrying out the above assays are as follows:

**T cells proliferation assay:** Sample collection, preparation and testing were performed as described in Gotsman et al., 2000. Antiviral Research, 48:17-26 and Akbar et al. Immunology 1993; 78: 468-475 as follows:
Peripheral blood mononuclear cells (PBMC) were isolated by Ficoll gradient separation. 1 X 10⁵ cells in 100uL RPMI 10% FCS were added to 4 triplicates of wells (1-4) on two separate plates - A (triplicates 1 and 2) and B (triplicates 3 and 4). In plate A, triplicate 1 wells contained cells and medium alone; triplicate 2 wells contained cells, HCV NS3 antigen (10 µg per well) and medium; In plate 2 - triplicate 3 contained cells and medium; triplicate 4 contained cells + PHA (2.5 µg/mL)+ medium. Two days (plate B) and 5 days (plate A) later, methyl-H³ thymidine (Amersham Pharmacia, GB) was added to the wells (1 µCi/mL). Cells were incubated for 18 hours then frozen, defrozen and harvested. Data was given as mean stimulation indices (SI) of triplicates ± SEM, calculated from the ratios of incorporated radioactivities of T cell cultures expressed as counts per minute (cpm) in the presence or absence of antigen. The results of this assay are shown in Figure 1.

**IFN-γ ELISPOT assay:** HCV IFN-γ spot forming cells (SPC) were determined using an HCV-specific ELISPOT assay (Mabtech, Nacka, Sweden) as described in Gotsman et al., op. cit and Akbar et al. op. cit. In brief, 96 well filtration plates coated with high protein binding hydrophobic PVDF membrane (polyvinylidene disulfide) were used (Millipore Corp., Bedford, MA, USA). Plates were coated using anti-IFN-γ coating antibody (15 mg/ml, Mabtech, Nacka, Sweden) for 24 hours at 40°C. Peripheral blood mononuclear cells (PBMC) were isolated by Ficoll gradient separation. Cells were cultured in 96 well plates (1x10⁵ cells/well) with RPMI 1640 and 10% FCS. Three triplicates were prepared with HCV NS3 (A), phytohemagglutinin (2.5 µg/ml) (B), or RPMI without antigen. Plates were incubated for 48 hours at 37°C and 5% CO₂. Following washing, dilute biotinylated antibodies (7-B6-1-biotin, Mabtech, Nacka, Sweden) were added in filtered PBS with 0.5% FCS to 1 µg/ml, in total volume of 100 µl/well. Plates were incubated for 3 hours at room temperature. Following washing, 100 ml of streptavidin-alkaline phosphatase was added and incubated for 90 minutes at room temperature. After washing, substrate was added (BCIP/NBT, BioRad, Richmond, USA) for 30 minutes until dark red purple spots emerged. After washing and drying, dark spots, reflecting IFN-γ-secreting clones were counted with a dissection microscope by 2 independent investigators. Results are expressed as means of triplicates IFN-γ secreting cells per 10⁵ lymphocytes after subtraction of mean spots from wells without viral antigens. The results of this assay are shown in Figure 2.

**IL-10 ELISPOT assay:** This assay was performed as described above for IFN-γ except that anti IL-10 coating antibody and anti IL-10 (second epitope) biotinylated antibodies were used. The results of this assay are shown in Figure 3.

**IFN-γ and IL4 serum level measurements:** These serum levels were measured by a "sandwich" ELISA, using Genzyme Diagnostics kits (Genzyme Diagnostics, MA, USA) according to the manufacturer's instructions. The results of these assays are shown in Figures 4 and 5.

**Flow cytometry analysis for NKT lymphocytes in peripheral blood:** Peripheral blood mononuclear cells (PBMC) were isolated by Ficoll gradient separation. Immediately after isolation, duplicates of 2-5 X 10⁴ cells/500 µL PBS were put into Falcon 2052 tubes, incubated with 4 ml of 1% BSA for 10 minutes, and centrifuged 1400 rpm for 5 minutes. Cells were resuspended in 10 µL FCS with 1:20 FITC anti human CD3 and CD56 antibodies (Pharmingen, USA) and mixed every 10 minutes for 30 minutes. Cells were washed twice in 1% BSA, and kept in 4ºC until reading. Analytical cell sorting was performed on 1 X 10⁴ cells from each group with a fluorescence activated cell sorter (FACSTAR Plus, Becton Dickinson). Only live cells were counted, and background fluorescence from non antibody-treated lymphocytes was subtracted from the levels obtained. Gates were set on forward and side scatters to exclude dead cells and red blood cells. Data was analyzed with the Consort 30 two-color contour plot program (Becton Dickinson, Oxnard, CA), or the CELLQuest Program 25.

These assays and figures demonstrate that the presence of an increased level of a metabolite has led to significant changes in the immune profile of these subjects. Surprisingly, when this condition was accompanied by another immune system challenge (HCV infection), there was significant impact on the immune profile of the HCV + subjects compared to the subjects that lacked elevation of the metabolite. Such differences manifested themselves in a variety of components of the immune system when the two conditions were superimposed. Thus one aspect of the present invention is to treat a subject such that enhanced levels of a metabolite are obtained in order to achieve at least one change in an immune component of said subject. Such a change may provide either enhanced immune response or reduced immune response or both. The changes may take place in different components in the immune system and may provide for different directionality in different components, i.e., some components may be enhanced and others may be reduced. For instance, increases in Th1 activities are frequently accompanied by reduction in Th2 responses and vice versa. It is also possible that both groups may proceed in the same direction. For instance, both IFN-γ (a marker for Th1 responsiveness) and IL-10 (a marker for Th2 responsiveness) both showed increases in Figures 2, 3 and 4. It is a further aspect of this invention that treatment of an infected individual particularly a subject with an HCV, HBV or HIV infection can be treated to achieve an elevated level of a metabolite in order to prevent further progression of the disease. In the case of HBV or HCV this progression would otherwise lead to fibrosis and destruction of the liver. In the case of HIV this progression leads to loss of immune competency.

The present invention can be carried out in various ways. For instance, a metabolite or a reagent that affects the level of such a metabolite can be introduced into a subject by intravenous means, intra-muscular means, subcutaneous means intra-peritoneal means or by oral means. Alternatively, treatment can proceed by an ex vivo procedure that includes removing cells from a subject and treating such cells with a metabolite and reintroducing the cells back into the patient. Examples of cells that could be removed and treated in this way can include but not be limited to peripheral blood monocytes (PBMCs), dendritic cells, T-cells, NK cells, NKT cells, CD1d cells, either separately or in combination. Such ex vivo treatments can further include other treatments such as exposure to cytokines, growth factors, matrices, antigens or other factors that promote growth or immune responses.

Effective amounts of the metabolite or reagent introduced into the cells intermediary metabolites should depend upon the individual pharmokinetic properties of said compounds to achieve sufficient levels of said metabolite in said subject for the duration desired. Such a level of the metabolite could be above the normal level for a sufficient time to induce an immune response in the subject. For example, the metabolite level in a Gaucher subject can be considered to be a guide for such levels.

Intermediary metabolites, such as glucosylceramides, can be used in accordance with this invention to treat various diseases, including cancer, infectious diseases and any immune-mediated pathogenic condition. For example in the instance of small cell carcinoma of the lung, subjects can be treated by administration of glucosylceramides such that at least one component of the immune system is elevated to such an extent that a specific activation of the NKT cell population is effected. Under these conditions the immune response to the cancer will be altered in such a manner that the cancer cells will be turned over or destroyed or lead to be destroyed and the subject will enter remission or experience a significant diminution of the cancer. A comparable effect can also be achieved by removing NKT cells from the subject and exposing these cells to glucosylceramides *in vitro* under conditions that will permit the survival and growth of the cells. When these *ex vivo*-trained cells are transferred back into the subject these cells will direct an immune response that can lead to a remission of the cancer or a significant diminution of the cancer.

This effect can potentially be achieved using appropriate metabolite treatment either *in vivo* or ex vivo for any disease or condition that has a part of all its pathology based on immune responses of the subject. Such conditions could include HBV, HCV, HIV, and other virus infections where the pathogenesis is based on an immune mediated pathogenesis. The present invention can also be applied to management of cancers, where the immune response contributes to the pathogenesis. Treatment of diseases of autoimmune or immune mediated origin is also a subject of the present invention. These can include but not be limited to diabetes type 1, diabetes type II, rheumatoid arthritis, Crohn's disease, arteriosclerosis, ulcerative colitis, and others that would be apparent to those practitioners who are skilled in the art.

### Further Description of the Invention

In one embodiment, this invention provides a process for treating a disease in a mammalian subject, e.g., a human, in which an effective amount of a mammalian intermediary metabolite or reagent is administered to the subject. By doing so, the intracellular or extracellular or serum level of the metabolite in the subject is raised. The intermediary metabolite can comprise lipids or conjugated biomolecules. The latter can take the form of glycolipids, lipoproteins and glycoproteins other than antibodies, cytokines or hormones. Such glycolipids can, in turn, comprise a monosaccharide ceramide, e.g., glucosyl ceramide or galatosyl ceramide.

Administration of the intermediary metabolite or reagent, as described further below, can be carried out by conventional means known in the art, including intravenous means, intra-muscular means, subcutaneous means, intra-peritoneal means or oral means.

In terms of the diseases that can be treated in accordance with the present invention, these includes cancers, infections and immune dysfunctions. Infections can be varied and include those whose etiology is viral or bacterial in nature. Viral infections include, for example, HBV, HCV and HIV. Immune dysfunctions can take the form of auto-immune disorders, including any of the following: diabetes type I, diabetes type II, rheumatoid arthritis, Crohn's disease, arteriosclerosis and ulcerative colitis.

The present invention also provides a process for treating a disease in a mammalian subject, e.g., a human, in which an effective amount of a reagent is administered that increases the intracellular or extracellular or serum level of a mammalian intermediary metabolite in the subject, e.g., a human. As described previously, the intermediary metabolite can comprise lipids or conjugated biomolecules, e.g., glycolipids, lipoproteins and glycoproteins other than antibodies, cytokines or hormones. The glycolipids can comprise a monosaccharide ceramide. Preferred are glucosyl ceramide or galatosyl ceramide.

As in the case of the previously described process, administration can be carried out by a number of conventional means, including intravenous means, intra-muscular means, subcutaneous means, intra-peritoneal means and oral means.

Diseases that are amenable to the present process include cancers, infections (viral, e.g., HBV, HCV and HIV, or bacterial) and immune dysfunctions. The latter can comprise auto-immune disorders, notably, diabetes type I, diabetes type II, rheumatoid arthritis, Crohn's disease, arteriosclerosis and ulcerative colitis.

In the afore-described process, the reagent can increase the rate of production of the mammlian intermediary metabolite in the subject, or alternatively, decrease the rate of degradation or turnover of said mammalian intermediary metabolite in the subject.

Also provided by the present invention is an ex vivo process for treating a disease in a mammalian subject. In this process, cells are obtained from the subject and treated with an effective amount of a mammalian intermediary metabolite so as to raise the intracellular level of the metabolite in the cells. Thereafter, the treated cells are transferred back to the subject using conventional procedures, such as intravenous administration.

As described earlier, the intermediary metabolite can comprise lipids or conjugated biomolecules, the latter including glycolipids, lipoproteins and glycoproteins other than antibodies, cytokines and hormones. Useful glycolipids include monosaccharide ceramides, such as glucosyl ceramide and galatosyl ceramide.

Disease conditions that may be treated in accordance with this invention include, by way of example, cancers, infections and immune dysfunctions. These have been described in further detail above.

The cells which can be treated and transferred back to the subject are various in nature and can include, for example, peripheral blood monocytes (PBMCs), dendritic cells, T cells, stem cells, NK cells, NKT cells and CD1 d cells.

In another aspect, the present invention provides a process for treating a disease in a mammalian subject comprising the first step of obtaining cells from the subject; followed by treatment of the cells with an effective amount of a reagent that increases the intracellular level of a mammalian intermediary metabolite in the cells. After treatment, the cells are transferred back to the subject.

The intermediary metabolite has been described above and needs no further elaboration.

After ex vivo treatment, the cells are typically transferred back to the subject intravenously.

Description of various diseases and afflictions have been given above and are applicable to the present process at hand.

As described above, in carrying out this process, it may be desirable that the reagent increases the rate of production of the mammlian intermediary metabolite in the subject, or alternatively, the reagent decreases the rate of degradation or turnover of the mammalian intermediary metabolite in the subject.

Although described above, it should be mentioned that the cells obtained from the subject could comprise any of the following cells: peripheral blood monocytes (PBMCs), dendritic cells, T cells, stem cells, NK cells, NKT cells and CD1d cells.

Yet another aspect of the present invention is a process for treating a disease in a mammalian subject, e.g., a human, comprising the step of administering to the subject an effective amount of a mammalian metabolite so as to modulate or change at least one component in the immune system of the subject. Such an immune system component can comprise cellular, humoral or cytokine elements, and the modulation or change can be specific or non-specific.

The intermediary metabolite has been described earlier and requires no further elaboration here. As earlier described, administration can be carried out by conventional means including intravenous means, intra-muscular means, subcutaneous means, intra-peritoneal means and oral means.

The process at hand may be applied to the previously described disease conditions and will not be discussed further.

Specific embodiments of the invention are:
1. A process for treating a disease in a mammalian subject comprising administering to said subject an effective amount of a mammalian intermediary metabolite so as to raise the intracellular or extracellular or serum level of said metabolite in said subject.
2. The process of item 1, wherein said intermediary metabolite comprises lipids or conjugated biomolecules.
3. The process of item 2, wherein said conjugated biomolecules comprise glycolipids, lipoproteins and glycoproteins other than antibodies, cytokines or hormones.
4. The process of item 3, wherein said glycolipid comprises a monosaccharide ceramide.
5. The process of item 4, wherein said monosaccharide ceramide comprises a glucosyl ceramide and galatosyl ceramide.
6. The process of item 1, wherein said administering step is carried out by means comprising intravenous means, intramuscular means, subcutaneous means, intraperitoneal means or oral means.
7. The process of item 1, wherein said disease comprises cancer, an infection or immune dysfunction.
8. The process of item 7, wherein said infection is viral or bacterial.
9. The process of item 8, wherein said viral infection comprises HBV, HCV or HIV.
10. The process of item 7, wherein said immune dysfunction comprises diabetes type I, diabetes type II, rheumatoid arthritis, Crohn's disease, arteriosclerosis and ulcerative colitis.
11. The process of item 1, wherein said mammalian subject comprises a human.
12. A process for treating a disease in a mammalian subject comprising administering to said subject an effective amount of a reagent that increases the intracellular or extracellular or serum level of a mammalian intermediary metabolite in said subject.
13. The process of item 12, wherein said intermediary metabolite comprises lipids or conjugated biomolecules.
14. The process of item 13, wherein said conjugated biomolecules comprise glycolipids, lipoproteins and glycoproteins other than antibodies, cytokines or hormones.
15. The process of item 14, wherein said glycolipid comprises a monosaccharide ceramide.
16. The process of item 15, wherein said monosaccharide ceramide comprises a glucosyl ceramide or galactosyl ceramide.
17. The process of item 12, wherein said administering step is carried out by means comprising intravenous means, intra-muscular means, subcutaneous means, intraperitoneal means or oral means.
18. The process of item 12, wherein said disease comprises cancer, an infection or immune dysfunction.
19. The process of item 18, wherein said infection is viral or bacterial.
20. The process of item 19, wherein said viral infection comprises HBV, HCV or HIV.
21. The process of item 18, wherein said immune dysfunction comprises diabetes type I, diabetes type II, rheumatoid arthritis, Crohn's disease, arteriosclerosis and ulcerative colitis.
22. The process of item 12, wherein said reagent increases the rate of production of said mammalian intermediary metabolite in said subject.
23. The process of item 12, wherein said reagent decreases the rate of degradation or turnover of said mammalian intermediary metabolite in said subject.
24. The process of item 12, wherein said mammalian subject comprises a human.
25. A process for treating a disease in a mammalian subject comprising:
   a) obtaining cells from said subject;
   b) treating said cells with an effective amount of a mammalian intermediary metabolite so as to raise the intracellular level of said metabolite in said cells; and
   c) transferring said treated cells to said subject.
26. The process of item 25, wherein said intermediary metabolite comprises lipids or conjugated biomolecules.
27. The process of item 26, wherein said conjugated biomolecules comprise glycolipids, lipoproteins and glycoproteins other than antibodies, cytokines or hormones.
28. The process of item 27, wherein said glycolipid comprises a monosaccharide ceramide.
29. The process of item 28, wherein said monosaccharide ceramide comprises a glucosyl ceramide and galatosyl ceramide.
30. The process of item 25, wherein said transferring step is carried out by intravenous means.
31. The process of item 25, wherein said disease comprises cancer, an infection or immune dysfunction.
32. The process of item 31, wherein said infection is viral or bacterial.
33. The process of item 32, wherein said viral infection comprises HBV, HCV or HIV.
34. The process of item 31, wherein said immune dysfunction comprises diabetes type I, diabetes type II, rheumatoid arthritis, Crohn's disease, arteriosclerosis and ulcerative colitis.
35. The process of item 25, wherein cells obtained from said subject comprise peripheral blood monocytes (PBMCs), dendritic cells, T cells, stem cells, NK cells, NKT cells and CD1d cells.
36. The process of item 25, wherein said mammalian subject comprises a human.
37. A process for treating a disease in a mammalian subject comprising:
   a) obtaining cells from said subject;
   b) treating said cells with an effective amount of a reagent that increases the intracellular level of a mammalian intermediary metabolite in said cells; and
   c) transferring said treated cells to said subject.
38. The process of item 37, wherein said intermediary metabolite comprises lipids or conjugated biomolecules.
39. The process of item 38, wherein said conjugated biomolecules comprise glycolipids, lipoproteins and glycoproteins other than antibodies, cytokines or hormones.
40. The process of item 39, wherein said glycolipid comprises a monosaccharide ceramide.
41. The process of item 40, wherein said monosaccharide ceramide comprises a glucosyl ceramide and galatosyl ceramide.
42. The process of item 37, wherein said transferring step is carried out by intravenous means.
43. The process of item 37, wherein said disease comprises cancer, an infection or immune dysfunction.
44. The process of item 43, wherein said infection is viral or bacterial.
45. The process of item 44, wherein said viral infection comprises HBV, HCV or HIV.
46. The process of item 43, wherein said immune dysfunction comprises diabetes type I, diabetes type II, rheumatoid arthritis, Crohn's disease, arteriosclerosis and ulcerative colitis.
47. The process of item 37, wherein said reagent increases the rate of production of said mammlian intermediary metabolite in said subject.
48. The process of item 37, wherein said reagent decreases the rate of degradation or turnover of said mammalian intermediary metabolite in said subject.
49. The process of item 37, wherein cells obtained from said subject comprise peripheral blood monocytes (PBMCs), dendritic cells, T cells, stem cells, NK cells, NKT cells and CD1 d cells.
50. A process for treating a disease in a mammalian subject comprising administering to said subject an effective amount of a mammalian metabolite so as to modulate or change at least one component in the immune system of said subject.
51. The process of item 50, wherein said immune system component comprises cellular, humoral or cytokine elements.
52. The process of item 50, wherein said modulation or change is specific or non-specific.
53. The process of item 50, wherein said intermediary metabolite comprises lipids or conjugated biomolecules.
54. The process of item 53, wherein said conjugated biomolecules comprise glycolipids, lipoproteins and glycoproteins other than antibodies, cytokines or hormones.
55. The process of item 54, wherein said glycolipid comprises a monosaccharide ceramide.
56. The process of item 55, wherein said monosaccharide ceramide comprises a glucosyl ceramide and galatosyl ceramide.
57. The process of item 50, wherein said administering step is carried out by means comprising intravenous means, intra-muscular means, subcutaneous means, intraperitoneal means or oral means.
58. The process of item 50, wherein said disease comprises cancer, an infection or immune dysfunction.
59. The process of item 58, wherein said infection is viral or bacterial.
60. The process of item 59, wherein said viral infection comprises HBV, HCV or HIV.
61. The process of item 58, wherein said immune dysfunction comprises diabetes type I, diabetes type II, rheumatoid arthritis, Crohn's disease, arteriosclerosis and ulcerative colitis.
62. The process of item 50, wherein said mammalian subject comprises a human.

Many obvious variations will no doubt be suggested to those of ordinary skill in the art in light of the above detailed description and examples of the present invention. All such variations are fully embraced by the scope and spirit of the invention as more particularly defined in the claims that now follow.

Furthermore, specific embodiments of the invention are:
Use of an effective amount of a mammalian intermediary metabolite which raises the intracellular or extracellular or serum level of said metabolite in a subject for the preparation of a pharmaceutical composition for treating cancer, an infection or immune dysfunction in said mammalian subject.

Use of an effective amount of a reagent that increases the intracellular or extracellular or serum level of a mammalian intermediary metabolite in a subject for the preparation of a pharmaceutical composition for treating cancer, an infection or immune dysfunction in said mammalian subject.

Use according to item 1 or 2 wherein the treatment comprises the transfer of cells to said subject which have been obtained from said subject and treated with the intermediary metabolite or the reagent, respectively.

Use of an effective amount of a mammalian metabolite which modulates or changes at least one component in the immune system of a subject for the preparation of a pharmaceutical composition for treating cancer, an infection or immune dysfunction in said mammalian subject.

The use of item 4, wherein said immune system component comprises cellular, humoral or cytokine elements.

The use of item 4, wherein said modulation or change is specific or non-specific.

The use of anyone of items 1 to 6, wherein said intermediary metabolite comprises lipids or conjugated biomolecules.

The use of item 7, wherein said conjugated biomolecules comprise glycolipids, lipoproteins and glycoproteins other than antibodies, cytokines or hormones.

The use of item 8, wherein said glycolipid comprises a monosaccharide ceramide.

The use of item 9, wherein said monosaccharide ceramide comprises a glucosyl ceramide and galactosyl ceramide.

The use of item 1, 2 or 4 to 10, wherein said intermediary metabolite is to be administered by means comprising intravenous means, intramuscular means, subcutaneous means, intraperitoneal means or oral means.

The use of item 3, wherein said transferring step is to be carried out by intravenous means.

The use of anyone of items 1 to 12, wherein said infection is viral or bacterial.

The use of item 13, wherein said viral infection comprises HBV, HCV or HIV.

The use of anyone of items 1 to 12, wherein said immune dysfunction comprises diabetes type I, diabetes type II, rheumatoid arthritis, Crohn's disease, arteriosclerosis and ulcerative colitis.

The use of anyone of items 1 to 15, wherein said mammalian subject comprises a human.

The use of anyone of items 3 or 7 to 16, wherein cells obtained from said subject comprise peripheral blood monocytes (PBMCs), dendritic cells, T cells, stem cells, NK cells, NKT cells and CD 1d cells.

The use of item 3 or 4, wherein said reagent increases the rate of production of said mammalian intermediary metabolite in said subject.

The use of item 3 or 4, wherein said reagent decreases the rate of degradation or turnover of said mammalian intermediary metabolite in said subject.

## Claims

1. Use of an effective amount of glucocerebroside for the preparation of a pharmaceutical composition for treating cancer.

2. The use of claim 1, wherein the cancer is a small cell carcinoma of the lung.

3. Glucocerebroside for use in the treatment of cancer.

4. Glucocerebroside for use according to claim 3, wherein the cancer is a small cell carcinoma of the lung.
